# EUROPEAN PATENT SPECIFICATION

(11) **EP 1 586 277 B1**
(45) Date of publication and mention of the grant of the patent: **09.05.2007**
(21) Application number: 04460010.4
(22) Date of filing: 16.04.2004
(51) Int. Cl.: A61B 18/02

(54) **Cryoapplicator for locally cooling a surface**
Kryoapplikator zum lokalen Kühlen einer Oberfläche
Cryoapplicateur pour refroidir une surface localement

(43) Date of publication of application: 19.10.2005
(73) Proprietor: Brojek, Wieslaw, 05-082 Blizne Laszczynskiego k. Warszawy (PL)
(72) Inventor: Brojek, Wieslaw, 05-082 Blizne Laszczynskiego k. Warszawy (PL)
(74) Representative: Czyz, Zbigniew

(56) References cited:
- EP-A- 0 633 008
- DE-A- 19 548 652
- FR-A- 2 815 246

## Description

The present invention relates to a cryoapplicator for local cooling down of a surface with cold gas injection, particularly for cooling down the chosen part of a human body surface.

Apparatus for cooling down of a surface by evaporating a liquid or cold gas injection are known. The most commonly used cooling mediums are liquid nitrogen (LN2) with the boiling temperature of -196° C or carbon dioxide (CO₂) with the boiling temperature of -70° C. It is known that cryogenic therapy is an effective way of treating rheumatism disorders, contusions and burns, it is also a perfect supplement to all sorts of physiotherapy treatments. Cryoptherapy is one of the best-known physical methods of posttraumatic treatment. Examples describing methods of treatment with cold are described in the publication of prof. Z. Zagrobelny "Krioterapia miejscowa i ogólnoustrojowa (Local and systemic cryoptherapy)", published by CZELEJ 1997.

From the Polish patent description No. 289451 there is known a gas injection apparatus used in cryotherapy. The apparatus comprises a tank of liquid nitrogen, a gas generator, an elastic feeding conduit, a cryoapplicator created by twice bent conduit, which intake end and exhaust end are placed parallel with each other and which are directed towards each other. An intake end is rotationally connected with the housing of cryoapplicator and with an elastic feeding conduit, and exhaust end is connected by the means of rotational articulated joint with the nozzle part. The housing by the means of another rotational articulated joint is secured to the column of the device.

From the Polish utility model application No. 107872 there is known a portable device for cooling down a part of a human body surface with the vapors of the low-boiling liquids, particularly carbon dioxide. Device comprises a gas cylinder with air-trap and a cut-off valve, a pressurized elastic conduit, a protecting shield - diffuser, an expander, and a handle that is secured to the gas cylinder.

From the European patent description EP 0633008 it is known a device for cryotheraphy comprising a pressure regulator and an exhaust system that includes manually actuated valve controlled by an external lever, a chamber placed below the valve that leads to exhaust pipe of a small cross section area. Properly chosen diameter of said pipe leads to the speed of flow that helps to avoid creation of dry ice during the expansion of the gas. Said exhaust pipe is matched to the concentric protective sleeve to achieve required values of temperature, pressure and emission of the carbon dioxide reaching chosen part of human body surface. A concentric sleeve is placed around an exhaust pipe, said sleeve has in its upper part intake openings drawing air according to the Ventouri phenomenon. The amount of the air that is flowing in, and in a consequence temperature and pressure in the cooled area depends on the size of the openings and a diameter of concentric sleeve.

FR 2 815 246 shows another type of cryoapplicator on which the preamble of claim 1 is based.

All those solutions have a main disadvantage, which is the large size of the devices due to a need of physical separation of a handle from a mixing chamber and an exhaust nozzle. Use of the cryoapplicator is rendered difficult due to the said large size of the device. Separation of those elements is necessary because during the expansion phase a huge amount of cold is released in the mixing chamber and the exhaust nozzle. The temperature of the mixing chamber walls and exhaust nozzle during the expansion of the carbon dioxide reaches the level below -50 °C. In the above mentioned solutions to avoid frostbites to the operator's hands or the necessity of wearing protective gloves, the handle used to manipulate the cryoapplicator is placed further away from those elements. Pressure regulator, levered cutting off valve that cuts off the flow of the liquid cooling gas, middle stage chambers and a pipe that leads from the chamber, are placed in the handle of the device from the EP 0633008. Such construction tends to increase the total weight of the cryoapplicator and to reduce the freedom of movement of the tip of the cryoapplicator. Another disadvantage related to the manipulating with the tip of the cryoapplicator is the lack of the possibility to instantaneously change the parameters of the cooling gas flowing out from the cryoapplicator. To obtain proper parameters of the gas directed to the chosen part of the surface, it's necessary, according to the given diameter of the exhausting pipe of the cooling gas, to properly choose the size of the outer concentric sleeve with the air inlet openings. The parameters of the cooling medium at the exhaust of the applicator can change due to the micro impurities getting at the liquid gas feeding system in the form of the single element or due to slow but constant settling of the impurities, due to wearing out of the cryoapplicator tip of the exhaust pipe caused by erosion, due to the change of the gas container temperature and due to change of the pressure in the gas container caused by lowering the level of the liquid gas.

The aim of the invention is providing a cryoapplicator that is free from the above mentioned problems in the description of the state of the art, particularly the aim of the invention is to provide a cryoapplicator that has unique features of monitoring and regulation of the parameters of the cooling medium during the treatment. The aim of the invention is simplifying the feeding system that leads to reducing weight and size of the cryoapplicator.

The invention is defined in claim 1.

Preferably the cryoapplicator is characterized in that a temperature sensor is placed inside the mixing chamber and an indicator of the exhaust gas temperature is placed in the handle.

Preferably the cryoapplicator is characterized in that between the grip surface being the handle and in external surface of the mixing chamber isolating and centering inserts are placed, preferably the inserts are made of thermo isolating material.

The cryoapplicator according to the invention has a grip surface that is effectively isolated from a very cold mixing chamber, and further has an ability to continuously regulate the temperature of the cooling medium. The continuous regulation of the cooling medium flow intensity during treatment in a longer time makes possible to keep stable and optimum parameters of cooling down a surface.

Another advantage of the embodiment of the present invention is the possibility of placing the control valve, that controls the liquid gas flow in the feeding system of the cryoapplicator, not in the handle. This arrangement allows to reduce the weight and size of the cryoapplicator and enables a usage of a standard valve designed to industrial applications. In the result the substantive increase of the reliability of the feeding control system of the cryoapplicator has been achieved.

The increased usability of the cryoapplicator during a cooling down treatment depends not only on the cooling medium monitoring and control system, but also on its weight and size, that allows the operator running the cooling down treatment to manipulate freely with the device during the treatment.

The cryoapplicator is connected with an elastic feeding conduit via fast release connection that allows quick replacements of the cryoapplicators.

The present invention has been disclosed in the preferred embodiment in the drawing where fig. 1 shows a cryoapplicator with air inlet from the side of the mouthpiece in the cross section, fig. **2** shows a cryoapplicator with an air inlet from the side of the mouthpiece in the cross section with the body in a partial view, fig. **3** shows a cryoapplicator with another version of the adjustable membrane in a half-view half-section, fig. **4** shows a isolating-centering profile in the cross section, fig. **5** shows another isolating-centering profile in the cross section, fig. **6** shows yet another isolating-centering profile in the cross section.

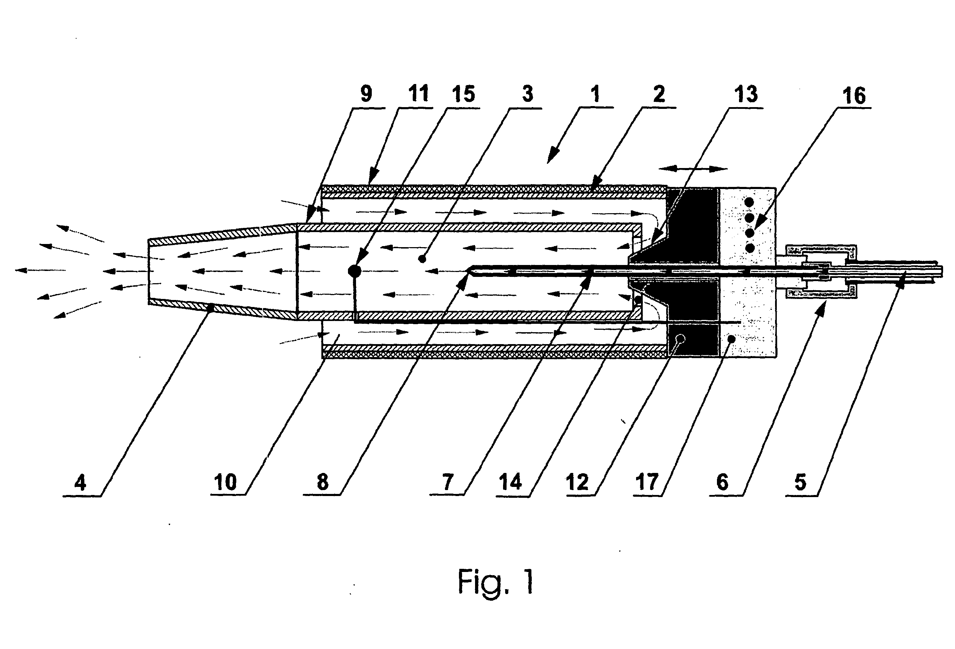

With reference to the fig. 1 cryoapplicator 1 according to the invention comprises a handle 2 that is a grip surface, in which the mixing chamber 3, that is provided with an intake mouthpiece 4, is placed. Liquid gas is fed to the mixing chamber 3 from the feeding system via a feeding conduit 5 that ends with a connector 6, pipe 7 and an expansion tip 8. Feeding line 5 and pipe 7 have an inner diameter from 1 to 4 mm, the expansion tip 8 is narrower part of the pipe 7 with an inner diameter from 0,1 to 0,45 mm depending on the size of the body surface to be cooled with the use of the cryoapplicator. The outside surface 9 of the mixing chamber 3 creates with surrounding handle 2 an air inlet channel 10 leading to the mixing chamber 3. The inlet mouthpiece 4 extends from the handle 2 allowing effective separation of the cold gas stream directed to the cooled down surface of a human body from the warm air flowing into the cryoapplicator 1. Furthermore, isolation of the handle 2 is thin isolation layer 11 of for example hard foamed polystyrene, said layer is paced between the edge of the inlet channel 10 and regulating ring 13. In the inlet channel 10, the air that is flowing into the cryoapplicator 1 is initially cooled down and is partially devoided from water while flushing an external surface 9 of the mixing chamber 3. Inlet channel 10 is also a dynamic isolation of the mixing chamber 3 from the handle 2. The expanded gas mixed with a proper amount of the air supplied via inlet channel 10 escapes through the outlet mouthpiece 4. Inlet channel 10 is divided by a membrane regulating the air flow to the mixing chamber 3. Regulated membrane creates a conical piece 13 of the regulating ring 12, that is centrally provided inside the pipe 7 and covers the opening 14 connecting inlet channel 10 with the mixing chamber 3. The regulation is realized through covering and uncovering the opening 14 connecting inlet channel 10 with the mixing chamber 3, i.e. through increase or decrease of the dumping of the air flow. Inside the mixing chamber 3 there is placed a temperature sensor 15, indicator of the exhaust gas temperature 16 is placed in the body 17 that creates an end part of the handle 2; an indicator of the exhaust gas temperature 16 is a multipoint indicator.

In the another not shown embodiment an accessory isolating layer consists of an electric heater that is placed between the handle 2 and the mixing chamber 3.

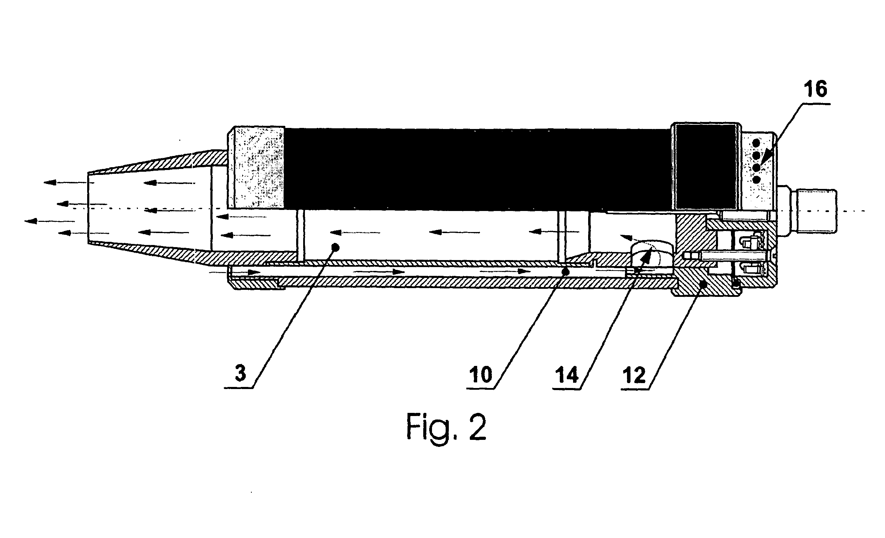

As shown in the fig. **2,** regulated membrane is made in the form of centric part of the regulating ring 12 in the shape of the cylinder closing the mixing chamber 3 from the back side. The cylinder covers logistical inlet opening 14' that connects inlet channel 10 with the mixing chamber 3. The regulation is realized through covering and uncovering the opening 14' connecting inlet channel 10 with the mixing chamber 3.

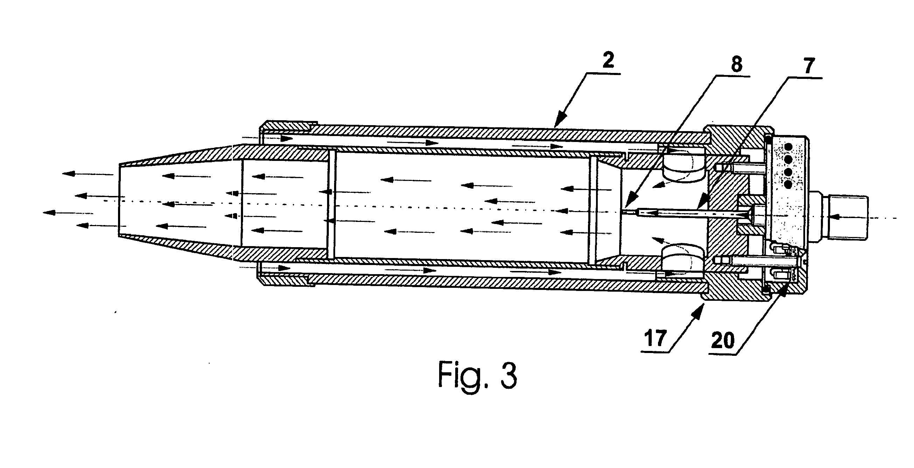

In the embodiment shown in the fig. **3,** pipe 7 ends with the capillary tube with length from 1 to 5 mm, that creates the expansion tip 8, inside the body 17 that creates an end of the cryoapplicator handle there is placed a printed circuit-board 20 with an electronic circuit for the temperature measurement

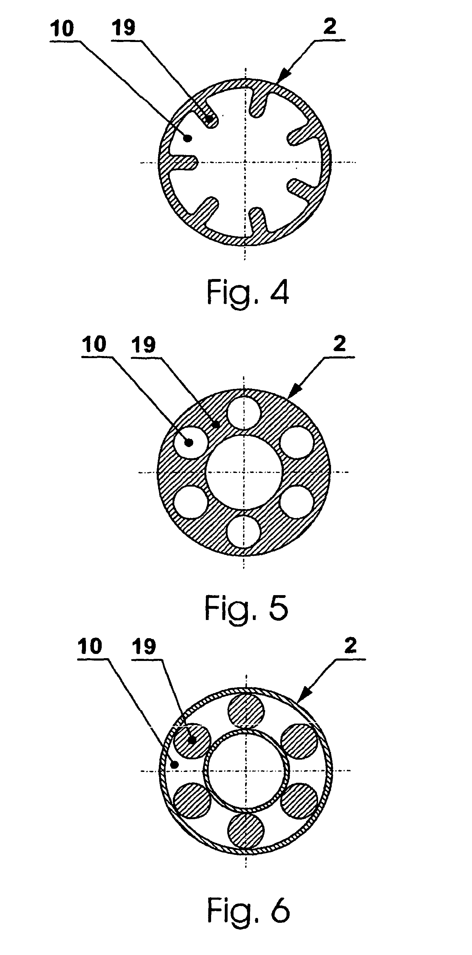

In the embodiment shown in the fig. **4** isolating and centering profile 19 created as one part of the handle 2, is shown. Inlet channels 10 are created by projections extending from the inner wall of the handle 2.

In the embodiment in the fig. **5** an isolating and centering profile 19' of the thermo isolating material in the form of a sleeve in which walls inlet channels 10' are placed, is shown.

In the embodiment in the fig. **6** isolating and centering profiles 19" in the form of the cylinders of the thermo isolating material, is shown. Said profiles are placed around external surface 9 of the mixing chamber 3 and create parallel inlet channels 10". It's possible to place the profiles in the spiral way that would create turbulent air flow entering the mixing chamber 3.

In yet another embodiment (not shown) a centering profile 19 is created as a part of the mixing chamber 3 and inlet channels 10 are created by projections extending from the external wall of the mixing chamber 3.

The handle 2 of the cryoapplictor can be made in another form than a cylinder so the housing can have square or oval cross section. Outlet mouthpiece 4 does not necessary need to be concentric with the mixing chamber and handle 2, and its shape can be similar to the widening flattened funnel. A handle 2, mixing chamber 3 and mouthpiece 4 can be made of metal; however for this purpose other suitable materials such as thermoplastic and thermosetting plastics can be used. Described embodiments do not cover all possible embodiments of the housings and centering profiles.

In an alternative but not illustrated embodiment mouthpiece 4 does not project from the grip surface 2, but this surface is provided with inlet louvers placed in a small distance from the mouth of the mouthpiece 4. Other placing of the louvers is also possible, said placing guarantees effective separation of the cold gas stream directed to the cooled down surface from warm air flowing into the cryoapplicator 1.

In another illustrated embodiment, temperature indicator is a tape indicator, in yet another it is a liquid crystal display, and the measurement of the cooled down surface temperature is realized remotely, it's also possible to make direct measurement.

In all above described embodiments of the present invention operator can precisely cool down even difficult reachable parts of a human body surface with the use of handheld cryoapplicator, without the risk of contact with very cold elements of the device.

The invention has an application in cryotherapy, but other applications in other fields can not be excluded, for example it can be used to create thermocompression bondings during assembling different parts of the machinery.

## Claims

1. A cryoapplicator (1) for locally cooling down of a surface by expansion of liquid gas, comprising a handle (2) with a grip surface, which contains a mixing chamber (3) which is fed with liquid gas and an adequate amount of air controlled by an intake valve, **characterized in that** between handle (2), and mixing chamber (3) there is placed an air inlet conduit (10), wherein the inlet conduit is located between the internal surface of the handle (2) and external surface (9) of the mixing chamber (3).

2. A cryoapplicator according to claim 1, **characterized in that** a temperature sensor (15) is placed inside the mixing chamber (3).

3. A cryoapplicator according to claim 1, **characterized in that** an indicator (16) of the exhaust gas temperature is placed in the handle (2).

4. A cryoapplicator according to claim 1, **characterized in that** between the handle (2) and the external surface (9) of the mixing chamber isolating and centering inserts (19, 19', 19") are placed.

5. A cryoapplicator according to claim 4, **characterized in that** the isolating and centering inserts (19, 19', 19") are made of thermo isolating material.

## Patentansprüche

1. Kryoapplikator zum lokalen Abkühlen einer Oberfläche durch Expansion eines flüssigen Gases, wobei der Kryoapplikator einen eine Grifffläche aufweisenden Handgriff (2) hat, der eine Mischkammer (3) enthält, die mit flüssigem Gas und einer ausreichenden Luftmenge gesteuert durch ein Einlassventil beschickt wird, **dadurch gekennzeichnet, dass** zwischen dem Handgriff (2) und der Mischkammer (3) eine Lufteinlassleitung (10) angeordnet ist, die sich zwischen der Innenfläche des Handgriffs (2) und der Außenfläche (9) der Mischkammer (3) befindet.

2. Kryoapplikator nach Anspruch 1, **dadurch gekennzeichnet, dass** in der Mischkammer (3) ein Temperatursensor (15) angeordnet ist.

3. Kryoapplikator nach Anspruch 1, **dadurch gekennzeichnet, dass** in dem Handgriff (2) ein Indikator (16) für die Auslassgastemperatur angeordnet ist.

4. Kryoapplikator nach Anspruch 1, **dadurch gekennzeichnet, dass** zwischen dem Handgriff (2) und der Außenfläche (9) der Mischkammer isolierende und zentrierende Einsätze (19, 19', 19") angeordnet sind.

5. Kryoapplikator nach Anspruch 4, **dadurch gekennzeichnet, dass** die isolierenden und zentrierenden Einsätze (19, 19', 19") aus einem Wärmeisoliermaterial hergestellt sind.

## Revendications

1. Cryoapplicateur (1) pour refroidissement local d'une surface par expansion de gaz liquide, comprenant une poignée (2) présentant une surface de préhension, qui contient une chambre de mélange (3) qui est alimentée par le gaz liquide et une quantité appropriée d'air contrôlé par une soupape d'admission, **caractérisé en ce que**, entre la poignée (2) et la chambre de mélange (3) est placé un conduit d'admission d'air (10), le conduit d'admission d'air étant situé entre la surface interne de la poignée (2) et la surface externe (9) de la chambre de mélange (3).

2. Cryoapplicateur selon la revendication 1, **caractérisé en ce qu'**un capteur de température (15) est placé à l'intérieur de la chambre de mélange (3).

3. Cryocapteur selon la revendication 1, **caractérisé en ce qu'**un indicateur (16) de la température de gaz d'échappement est placé dans la poignée (2).

4. Cryocapteur selon la revendication 1, **caractérisé en ce que**, entre la poignée (2) et la surface externe (9) de la chambre de mélange, des inserts d'isolement et de centrage (19, 19', 19") sont placés.

5. Cryocapteur selon la revendication 4, **caractérisé en ce que** les inserts d'isolement et de centrage (19, 19', 19") sont constitués de matériau d'isolation thermique.
